(19) Europäisches Patentamt
European Patent Office
Office européen des brevets

(11) Numéro de publication: **0 266 246**
**A1**

(12) **DEMANDE DE BREVET EUROPEEN**

(21) Numéro de dépôt: **87402222.1**

(22) Date de dépôt: **07.10.87**

(51) Int. Cl.[4]: **C 07 D 471/04**
A 61 K 31/44
//(C07D471/04,235:00,221:00)

(30) Priorité: **08.10.86 FR 8613995**

(43) Date de publication de la demande:
**04.05.88 Bulletin 88/18**

(84) Etats contractants désignés:
**AT BE CH DE ES FR GB GR IT LI LU NL SE**

(71) Demandeur: **SYNTHELABO**
**58 rue de la Glacière**
**F-75013 Paris (FR)**

(72) Inventeur: **Manoury, Philippe**
**L'Orée de Verrières 38, Avenue des Vaupépins**
**F-91370 Verrières le Buisson (FR)**

**Binet, Jean**
**12, Hameau de la Gondole**
**F-91650 Breuillet (FR)**

**Dewitte, Elisabeth**
**38, Avenue d'Orgeval**
**F-95210 Saint Gratien (FR)**

(74) Mandataire: **Thouret-Lemaitre, Elisabeth et al**
**Service Brevets - SYNTHELABO 58, rue de la Glacière**
**F-75621 Paris Cédex 13 (FR)**

(54) **Dérivés d'imidazo[4,5-b]pyridinone-2, leur préparation et leur application en thérapeutique.**

(57) Dérivés d'imidazo[4,5-b]pyridinone-2 répondant à la formule (I)

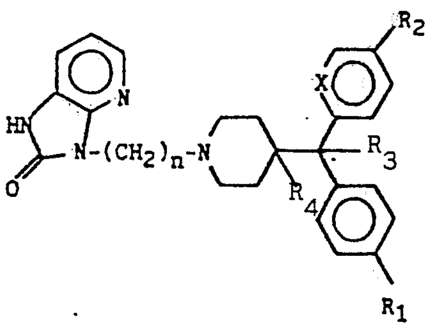

(I)

dans laquelle
n est 2, 3 ou 4,
X est CH ou N,
$R_1$ et $R_2$ représentent chacun, indépendamment l'un de l'autre, un atome d'hydrogène ou d'halogène ou un radical ($C_{1-4}$)alcoxy et,
soit $R_3$ est H ou OH et $R_4$ est H, soit $R_3$ et $R_4$ forment une liaison.
ainsi que leurs énantiomères et leurs sels d'addition aux acides pharmaceutiquement acceptables.
Application en thérapeutique.

EP 0 266 246 A1

## Description

DERIVES D'IMIDAZO[4,5-b]PYRIDINONE-2, LEUR PREPARATION ET LEUR APPLICATION EN THERAPEUTIQUE

La présente invention a pour objet des dérivés d'imidazo [4,5-b]pyridinone-2, leur préparation et leur application en thérapeutique.

Les composés de l'invention répondent à la formule (I) donnée en annexe 1 dans laquelle
n est 2, 3 ou 4,
X est CH ou N,
$R_1$ et $R_2$ représentent chacun, indépendamment l'un de l'autre, un atome d'hydrogène ou d'halogène ou un radical ($C_{1-4}$)alcoxy et,
soit $R_3$ est H ou OH et $R_4$ est H, soit $R_3$ et $R_4$ forment une liaison.

Les sels d'addition que les composés (I) peuvent former avec les acides pharmaceutiquement acceptables font partie de l'invention.

Les composés peuvent donner naissance à des énantiomères, par exemple lorsque $R_1$ est différent de $R_2$ ou lorsque X est N, dans ce cas les énantiomères des composés (I) font partie de l'invention.

Selon l'invention on peut préparer les composés (I) selon les deux schémas réactionnels 1 et 2, donnés en annexes 1 et 2.

Selon le schéma réactionnel 1, on fait réagir un composé (II)
- soit avec un composé (III) pour obtenir le composé (IV) que l'on fait réagir avec de l'hydrazine pour obtenir le composé (VI),
- soit avec de l'acrylonitrile ou un composé Y-$(CH_2)_{n-1}$-CN dans lequel Y est un groupe labile, pour obtenir le composé (V) que l'on hydrogène, en présence de nickel de Raney, en composé (VI).

On fait alors réagir celui-ci avec la chloro-2 nitro-3 pyridine, en présence de carbonate de potassium, dans de l'étha nol, pour obtenir le composé (VII) que l'on hydrogène en composé (VIII) ; enfin on fait réagir ce composé (VIII) avec du chloroformiate d'éthyle ou du pyrocarbonate d'éthyle dans du toluène pour obtenir le composé (I). Alternativement, on peut faire réagir l'intermédiaire (VIII) avec de l'urée entre 100 et 180°C ou avec du carbonyldiimidazole. Selon le schéma réactionnel 2, on estérifie le composé (IX) en composé (X) que l'on hydrogène en composé (XI), on fait réagir ce dernier avec de l'urée pour obtenir le composé (XII) que l'on hydrolyse en alcool (XIII), on transforme ce composé en dérivé de l'alcool (XIV), dans lequel Y est un groupe labile, et finalement on fait réagir le composé (XIV) avec un composé (II) pour obtenir le composé (I).

Les schémas réactionnels 1 et 2 sont illustrés dans les exemples suivants.

Certains des composés (II) sont décrits dans le brevet américain 2804222 et dans la littérature par Duncan et al., J. Med. Chem. 13, 1, 1970.

Les composés (II) dans lesquels $Ar_1$ ou $Ar_2$ représente un radical pyridinyle ou phényle peuvent être préparés selon le schéma réactionnel 3 donné en annexe 3 : on fait réagir un composé (XV) avec un composé organométallique (XVI) puis on hydrolyse ou on hydrogénolyse le composé obtenu (XVII) pour arriver au composé (II).

Les composés (I) dans lesquels $R_3 = R_4 = H$ peuvent être également obtenus par hydrogénolyse des composés (I) dans lesquels $R_3 = OH$ et $R_4 = H$ ou hydrogénation des composés (I) dans lesquels $R_3$ et $R_4$ représentent ensemble une liaison.

Les exemples suivants illustrent l'invention. Les analyses et les spectres IR et RMN confirment la structure des composés.

Exemple 1 [[Hydroxydiphénylméthyl)-4 pipéridinyl-1]-2 éthyl]-3 dihydro-1,3 2H-imidazo[4,5-b]pyridinone-2.

1.1. [[(Hydroxydiphénylméthyl)-4 pipéridinyl-1]-2 éthyl]-2 1H-isoindole-dione-1,3.

On porte, à la température du reflux, pendant 5 heures, un mélange de 15 g (0,056 mole) de α, α-diphényl-pipéridine-4 méthanol, de 14,5 g (0,056 mole) de (bromo-2 éthyl)-2 1H-isoindole-dione-1,3 et de 6,7 g (0,063 mole) de carbonate de sodium dans 150 ml de méthylisobutylcétone.

Après évaporation du solvant on reprend le résidu avec de l'eau et du chloroforme. On lave la phase organique avec de l'eau, la sèche, la filtre et l'évapore. On obtient une huile qui cristallise après purification sur colonne de silice. On obtient un produit fondant à 166-169°C.

1.2. (amino-2 éthyl)-1 α,α-diphényl pipéridine-4-méthanol.

On agite 12 h, à la température ambiante, une solution de 11,7 g (0,026 mole) du dérivé précédent dans 200 ml de méthanol contenant 1,3 ml d'hydrazine. On évapore à sec, reprend le résidu avec de l'eau et acidifie avec de l'acide chlorhydrique. On filtre l'insoluble puis extrait avec du chlorure de méthylène.
On alcalinise la phase aqueuse, extrait avec du chlorure de méthylène. On lave la phase organique avec de l'eau, sèche, filtre et évapore.
On obtient le produit blanc fondant à 164-166°C.

1.3. [[(nitro-3 pyridinyl-2)amino]-2 éthyl]-1 α,α-diphényl pipéridine-4-méthanol.

On porte au reflux 11,9 g (0,04 mole) du produit précédent avec 6,4 g (0,04 mole) de chloro-2 nitro-3 pyridine et 5,8 g (0,042 mole) de carbonate de potassium dans 200 ml d'éthanol pendant 12 h.
On évapore le mélange, reprend le résidu d'évaporation avec de l'eau et de l'éther. On lave la phase éthérée avec de l'eau, sèche, filtre et évapore.
On obtient une huile jaune que l'on utilise brute dans l'é tape suivante.

1.4. [[(amino-3 pyridinyl-2) amino]-2 éthyl]-1 α,α-diphényl pipéridine-4-méthanol.

On hydrogène à la température ambiante, sous une pression d'hydrogène de 50 psi, en présence de 0,5 g d'oxyde de platine, 17,3 g (0,04 mole) du dérivé nitré précédent en solution dans 150 ml de méthanol.
Lorsque la réaction est terminée on filtre le catalyseur et évapore le filtrat.
On obtient une huile très colorée que l'on utilise brute pour la suite de la synthèse.

1.5. [[(Hydroxydiphénylméthyl)-4 pipéridinyl-1]-2 éthyl]-3 dihydro-1,3 2<u>H</u>-imidazo[4,5-<u>b</u>]pyridinone-2.

On introduit avec précaution 16,8 ml (0,114 mole) de pyrocarbonate d'éthyle dans une solution de 15,2 g (0,038 mole) de l'huile précédente dans 100 ml de toluène. On agite 2 h après la fin de l'addition puis évapore le toluène. On solubilise la gomme noire ainsi obtenue dans 100 ml d'éthanol ; on ajoute 1 g de sodium et porte au reflux 1 h.
On évapore ensuite le mélange et reprend la gomme résiduelle avec de l'eau. On neutralise avec 2,5 ml d'acide acétique, extrait avec du chlorure de méthylène. On lave la phase organique avec de l'eau, sèche,filtre et évapore.
On purifie le produit obtenu par chromatographie sur colonne de silice. On obtient le produit que l'on fait recristalliser dans de l'acétate d'éthyle. F = 213-217°C.

<u>Exemple 2</u>. [[[Hydroxy(fluoro-4 phényl) (pyridinyl-2)méthyl]-4 pipéridinyl-1]-3 propyl]-3 dihydro-1,3 2<u>H</u>-imidazo[4,5-<u>b</u>] pyridinone-2.

2.1. [[Hydroxy(fluoro-4 phényl) (pyridinyl-2)méthyl]-4 pipéridinyl-1]-3 propanenitrile.

On ajoute, goutte à goutte, 2,3 ml (0,035 mole) d'acryloni trile à une solution de 10 g (0,035 mole) de α-(fluoro-4 phényl) α-(pyridinyl-2) pipéridine-4-méthanol dans 60 ml d'éthanol et 1 ml de Triton B.
On laisse la solution 2 jours à la température ambiante. On évapore à sec, reprend le mélange résiduel avec de l'eau et du chlorure de méthylène. On lave la phase organique avec de l'eau, sèche, filtre et évapore.
On obtient le produit cristallisé. F = 45-48°C.

2.2. (amino-3 propyl)-1 α-(fluoro-4 phényl) α-(pyridinyl-2) pipéridine-4-méthanol.

On hydrogène dans un autoclave, sous une pression d'hydrogène de 60 kg/cm2, à une température de 70°C, 12,2 g (0,035 mole) du nitrile précédent en solution dans 70 ml d'éthanol ammoniacal, en présence de nickel de Raney.
Lorsque la réaction est terminée, on filtre le catalyseur et évapore le filtrat. On obtient une huile incolore utilisable sans autre purification pour l'étape suivante. Un échantillon est transformé en oxalate. F = 124-129°C.

2.3. [[Nitro-3 pyridinyl-2)amino]-3 propyl]-1 α-(fluoro-4 phényl) α-(pyridinyl-2) pipéridine-4-méthanol.

On prépare ce produit en utilisant la méthode décrite dans le paragraphe 1.3 à partir de 7,6 g (0,022 mole) du produit précédent et de 3,5 g (0,022 mole) de chloro-2 nitro-3 pyridine. On obtient le produit que l'on utilise brut dans la suite de la synthèse.

2.4. [[(amino-3 pyridinyl-2) amino]-3 propyl]-1 α-(fluoro-4 phényl) α-(pyridinyl-2) pipéridine-4-méthanol.

On utilise la méthode décrite dans le paragraphe 1.4 à partir de 5,6 g (0,012 mole) du dérivé précédent. On obtient une huile fortement colorée que l'on utilise brute dans l'étape suivante.

2.5. [[[Hydroxy(fluoro-4 phényl)(pyridinyl-2)méthyl]-4 pipéridinyl-1]-3 propyl]-3 dihydro-1,3 2<u>H</u>-imidazo[4,5-<u>b</u>]pyridinone-2.

On prépare le produit en utilisant la même méthode que celle qui est décrite dans le paragraphe 1.5, à partir de 5,2 g (0,012 mole) du produit précédent et de 7 ml de pyrocarbonate d'éthyle. On obtient le produit que l'on fait recristalliser dans de l'acétate d'éthyle. F = 140-144°C.

<u>Exemple 3</u>. [(Hydroxydiphénylméthyl)-4 pipéridinyl-1]-1 propyl-3]-3 dihydro-1,3 2<u>H</u>-imidazo[4,5-<u>b</u>]pyridinone-2.

3.1. Benzoate de [(nitro-3 pyridinyl-2)amino]-3 propyle-1.

On introduit, goutte à goutte, 70 ml de chlorure de benzoyle dans une solution de 77,8 g (0,04 mole) de [(nitro-3 pyridinyl-2)amino]-3 propanol (M. ISRAEL, N. TIROSH J. Med. Chem. 1973, 16, 520) dans 48,5 ml de pyridine et 800 ml de benzène. On agite le mélange 2 h, à la température ambiante puis filtre le précipité. On lave le filtrat avec de l'acide chlorhydrique N, puis avec de l'eau. On sèche,filtre et évapore. On obtient un

produit cristallisé.

3.2. Benzoate d'[(amino-3 pyridinyl-2)amino]-3 propyle-1.

On hydrogène, dans un appareil de Parr, à la température ambiante, sous une pression d'hydrogène de 50 psi, 30 g (0,1 mole) du dérivé nitré précédent en solution dans 300 ml de méthanol en présence de 2 g d'oxyde de platine.

Après traitement habituel, on obtient le produit très coloré que l'on utilise tel quel pour l'étape suivante.

3.3. Benzoate d'(hydroxy-1 propyl-3)-3 dihydro-1,3 2<u>H</u>-imidazo[4,5-<u>b</u>]pyridinone-2.

On chauffe à la température de reflux, pendant 24 h, 27,1 g (0,1 mole) du produit précédent avec 24,3 g (0,15 mole) de carbonyldiimidazole en solution dans 150 ml de chloroforme.

On refroidit le mélange, le lave avec de l'eau, sèche la phase organique, filtre et évapore. On chromatographie le produit obtenu sur une colonne de silice ; on obtient le produit que l'on fait recristalliser dans un mélange d'éther isopropylique et d'acétate d'éthyle. F = 145-148°C.

3.4. (Hydroxy-1 propyl-3)-3 dihydro-1,3 2<u>H</u>-imidazo[4,5-<u>b</u>]pyridinone-2.

On hydrolyse à 50-60°C, 18,2 g (0,061 mole) de l'ester précédent dans 300 ml de méthanol à 85 % et 8,5 g de potasse en pastilles. Lorsque la réaction est terminée, on évapore le mélange et extrait le résidu avec de la méthyléthylcétone au reflux. On évapore le solvant et obtient le produit que l'on fait recristalliser dans de la méthyléthylcétone.

(F = 160-163°C).

On prépare le chlorhydrate dans du méthanol par addition d'éther chlorhydrique. F = 186-190°C.

3.5. Chlorhydrate de (chloro-1 propyl-3)-3 dihydro-1,3 2<u>H</u>-imidazo[4,5-<u>b</u>]pyridinone-2.

On porte au reflux, pendant 4 h, une suspension de 5 g (0,021 mole) du produit précédent sous forme de chlorhydrate dans 100 ml de chlorure de thionyle et 0,3 ml de DMF.

On évapore le chlorure de thionyle en excès et reprend l'huile résiduelle avec de l'acétone. On obtient le produit sous forme solide.

3.6. [(Hydroxydiphénylméthyl)-4 pipéridinyl-1]-1 propyl-3]-3 dihydro-1,3 2<u>H</u>-imidazo[4,5-<u>b</u>]pyridinone-2.

On porte au reflux, pendant 5 h, 1,05 g (0,005 mole) du produit précédent, 1,3 g (0,005 mole) de α,α-diphényl-pipéridine-4 méthanol et 1 g de bicarbonate de sodium dans 50 ml d'éthanol.

On filtre les produits minéraux et évapore le filtrat. On solubilise le résidu dans du chloroforme, lave la phase organique avec de la soude N, puis avec de l'eau. On sèche, filtre et évapore. On purifie le produit par chromotagraphie sur colonne de silice. On obtient le produit que l'on fait recristalliser dans de la méthyléthylcétone. F = 190-192°C.

<u>Exemple 4</u>. [[[bis(fluoro-4 phényl)méthylène]-4 pipéridinyl-1]-3 propyl]-3 dihydro-1,3 2<u>H</u>-imidazo[4,5-b]pyridinone-2.

4.1. (Amino-3 propyl)-1 α,α-bis(fluoro-4 phényl)pipéridine-4-méthanol.

Ce produit est préparé de la manière décrite dans l'exemple 2.2 à partir de 18,2 g de [[bis(fluoro-4 phényl)hydroxyméthyl]-4 pipéridinyl-1]-3 propanenitrile.

4.2. [[[bis(fluoro-4 phényl)hydroxyméthyl]-4 pipéridinyl-1]-3 propyl]-3 dihydro-1,3 2<u>H</u>-imidazo[4,5-pyridinone-2.

Ce produit est synthétisé à partir du produit obtenu en 4.1. selon la méthode décrite dans l'exemple 2 (2.3. à 2.5.). F = 183-185°C.

4.3. On porte au reflux pendant 2 h, 1 g de [[bis(fluoro-4 phényl) hydroxyméthyl]-4 pipéridinyl-1]-3 propyl]-3 dihydro-1,3 2<u>H</u>-imidazo[4,5-b]pyridinone-2 en solution dans 50 ml d'acide chlorhydrique 6N.

On alcalinise le mélange réactionnel, extrait avec du chlorure de méthylène. Après traitement habituel, on obtient un produit que l'on purifie par chromatographie sur colonne de silice. On obtient le produit cristallisé. F = 163-165°C.

Tableau

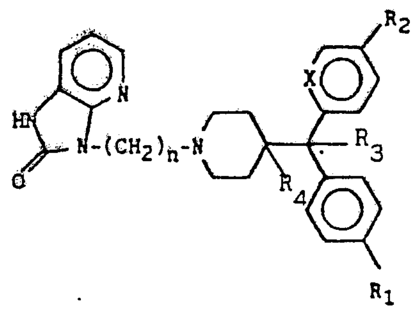

(I)

| Composé | n | X | $R_1$ | $R_2$ | $R_3$ | $R_4$ | F(°C) |
|---|---|---|---|---|---|---|---|
| 1 | 2 | CH | H | H | OH | H | 213-217 |
| 2 | 2 | CH | H | H | H | H | 228-229 |
| 3 | 3 | CH | H | H | OH | H | 190-192 |
| 4 | 3 | CH | F | F | OH | H | 183-185 |
| 5 | 3 | CH | F | F | liaison | | 163-165 |
| 6 | 3 | CH | F | H | OH | H | 182-188 |
| 7 | 3 | CH | Cl | F | OH | H | 138 |
| 8 | 3 | CH | $CH_3O$ | F | OH | H | 161-164 |
| 9 | 3 | N | F | H | OH | H | 140-144 |
| 10 | 4 | N | F | H | OH | H | 155 |

Les composés ont été soumis à divers essais pharmacologiques montrant leur activité antagoniste de l'histamine et de la sérotonine.

Activité in vivo : inflammation induite par l'histamine ou la sérotonine.

L'injection intraplantaire dans une des pattes postérieures du rat, d'histamine (200 µg) ou de sérotonine (1 µg), provoque un oedème mesuré, 1 h après l'injection, à l'aide d'un pléthysmomètre à mercure Ugo Basile. Les composés de l'invention, mis en suspension dans du tween en solution à 1 % dans de l'eau distillée, sont administrés p.o. (0,5 ml/100g) 1 h avant l'injection de l'agent inflammatoire.
Les $DA_{40}$ (dose qui diminue de 40 % le volume de l'oedème) sont mesurées.
Les composés de l'invention ont une $DA_{40}$ allant de 0,5 à 5 mg/kg lorsque l'agent inflammatoire est l'histamine.
Certains composés de l'invention sont actifs à une dose $DA_{40}$ allant de 0,2 à 2 mg/kg lorsque l'agent inflammatoire est la sérotonine.

Les composés de l'invention peuvent donc être utilisés comme antiallergiques, antiprurigineux pour le traitement des allergies respiratoires telles que rhinites, rhumes des foins, allergies cutanées telles que dermatites, urticaires, allergies oculaires, oedème de Quincke et manifestations allergiques diverses et pour le traitement de l'asthme.

Les dérivés de l'invention plus spécifiquement actifs comme antagonistes de la sérotonine peuvent être utilisés pour lutter contre certains effets indésirables de ce médiateur au niveau périphérique ou au niveau central. Ils sont destinés en particulier au traitement de la migraine.

L'invention comprend, par conséquent, toutes compositions pharmaceutiques renfermant les composés et/ou leurs sels comme principes actifs, en association avec tous excipients appropriés à leur administration, en particulier par voie orale ou parentérale. Les voies d'administration peuvent être les voies orale et parentérale. La posologie quotidienne peut aller de 5 à 200 mg.

Annexe 1

Schéma 1

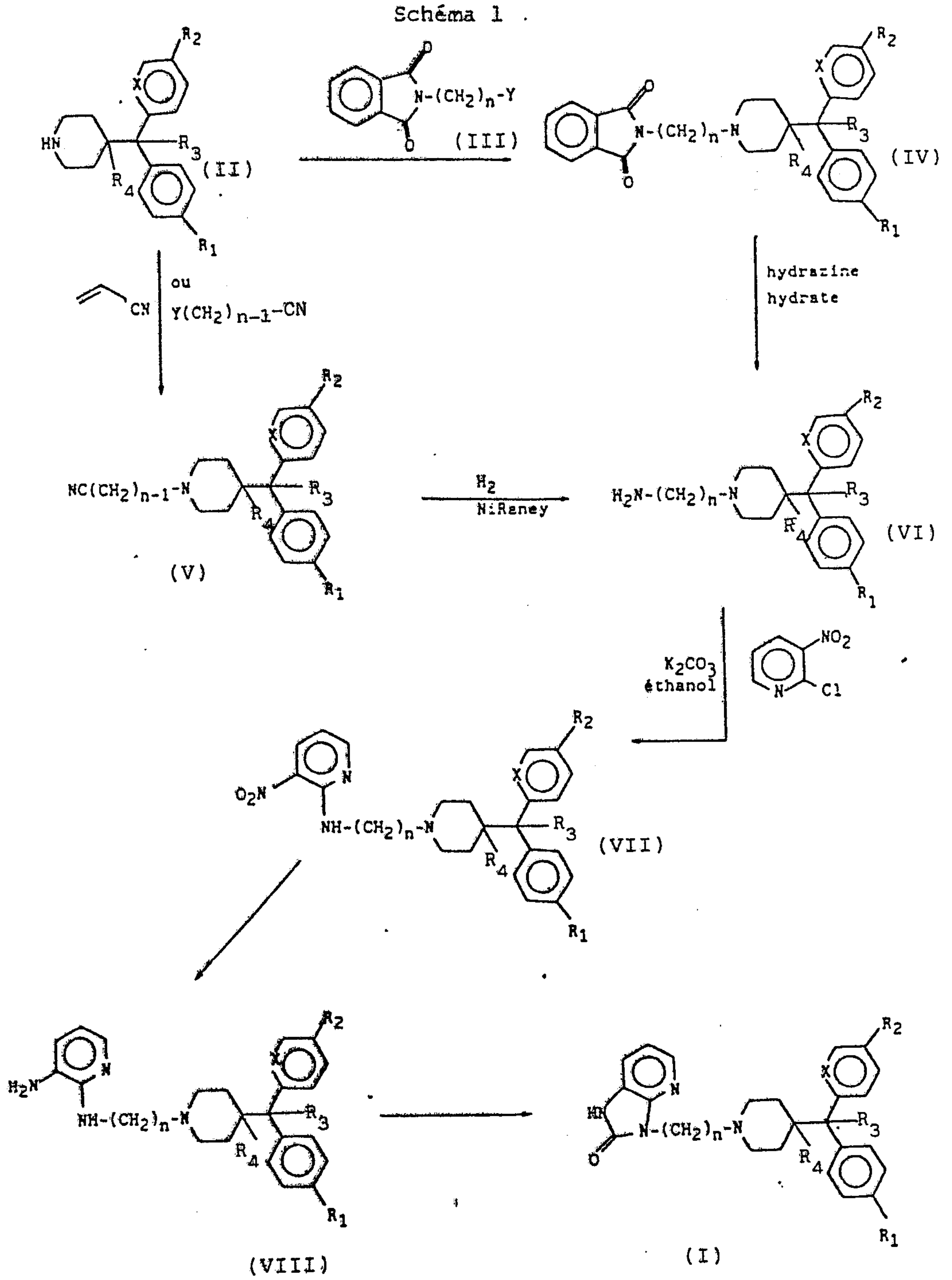

Annexe 2

Schéma 2

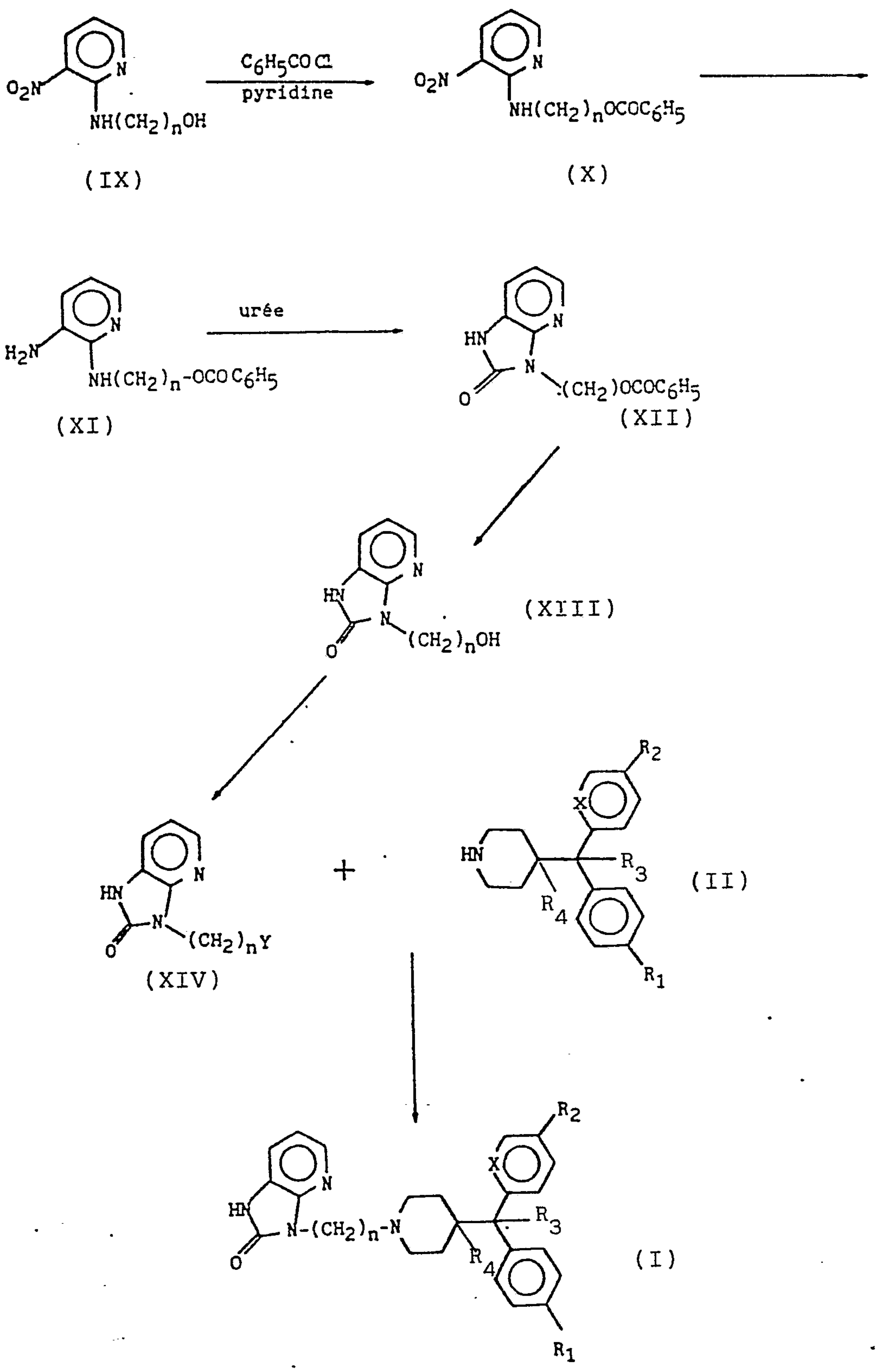


(IX) (X) (XI) (XII) (XIII) (XIV) (II) (I)

Annexe 3

Schéma 3

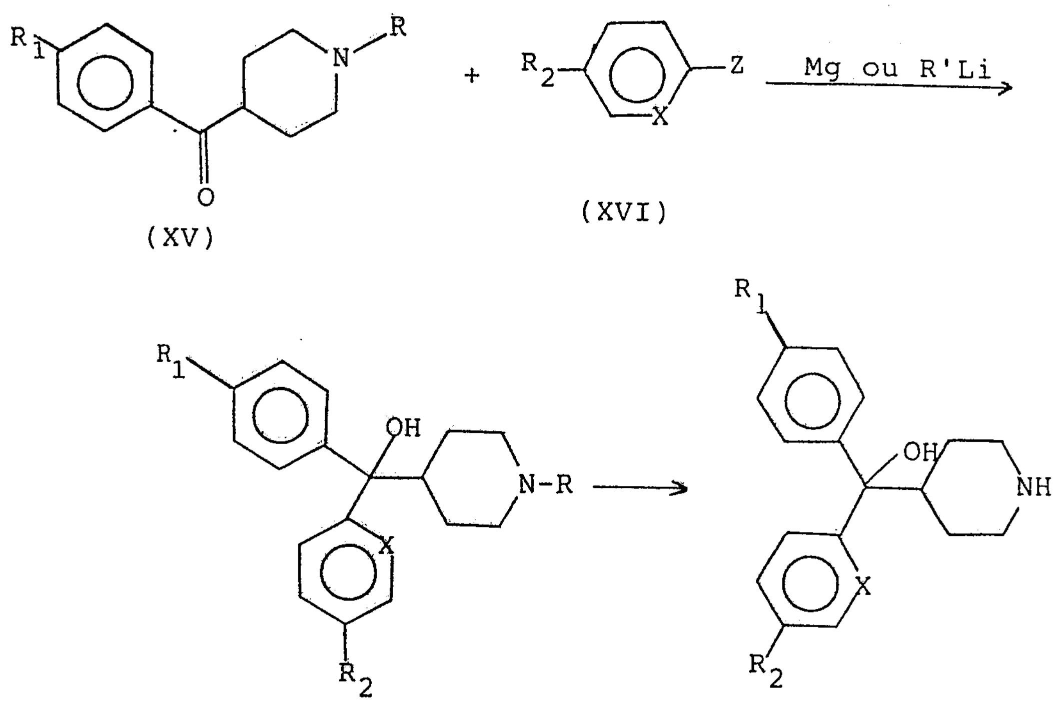


(XV)

(XVI)

(XVII)

(II)

**Revendications**

1. Dérivés d'imidazo[4,5-b]pyridinone-2 répondant à la formule (I)

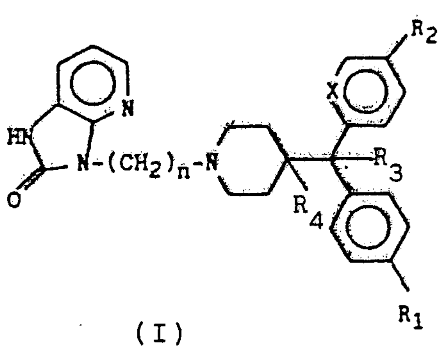

(I)

dans laquelle
n est 2, 3 ou 4,
X est CH ou N,
$R_1$ et $R_2$ représentent chacun, indépendamment l'un de l'autre, un atome d'hydrogène ou d'halogène ou un radical $(C_{1-4})$alcoxy et,
soit $R_3$ est H ou OH et $R_4$ est H, soit $R_3$ et $R_4$ forment une liaison.
ainsi que leurs énantiomères et leurs sels d'addition aux acides pharmaceutiquement acceptables.

2. Procédé de préparation des composés selon la revendication 1, procédé caractérisé en ce que l'on fait réagir un composé (II)

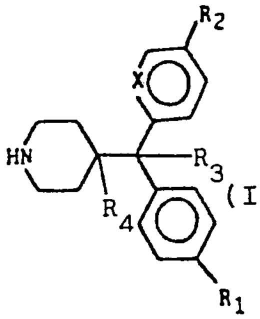

(II)

- soit avec un composé (III)

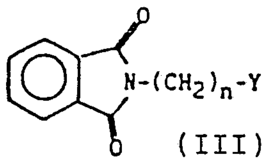

(III)

pour obtenir le composé (IV)

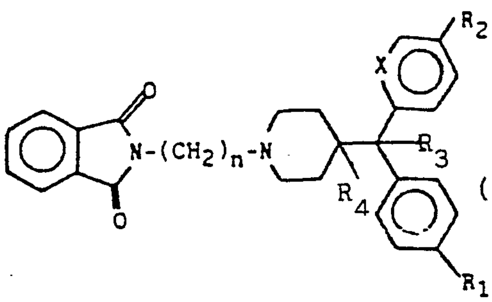

(IV)

que l'on fait réagir avec de l'hydrazine pour obtenir le composé (VI),
- soit avec de l'acrylonitrile ou un composé Y-$(CH_2)_{n-1}$-CN dans lequel Y est un groupe labile, pour obtenir le composé (V)

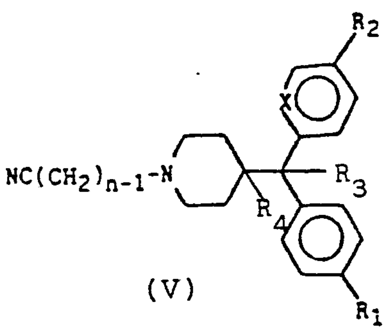

(V)

que l'on hydrogène, en présence de nickel de Raney, en composé (VI).

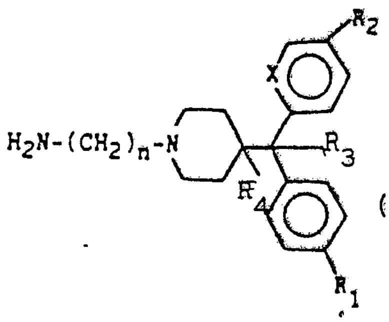

(VI)

On fait alors réagir celui-ci avec la chloro-2 nitro-3 pyridine, en présence de carbonate de potassium, dans de l'éthanol, pour obtenir le composé (VII)

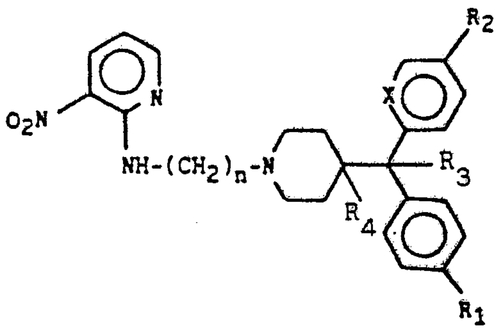

(VII)

que l'on hydrogène en composé (VIII)

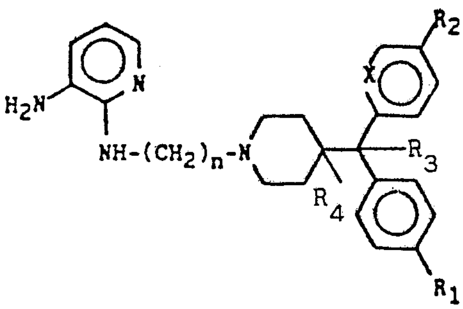

(VIII)

et enfin on fait réagir ce composé, par exemple, avec du pyrocarbonate d'éthyle dans du toluène pour obtenir le composé (I).

3. Procédé de préparation des composés selon la revendication 1, procédé caractérisé en ce que l'on estérifie le composé (IX)

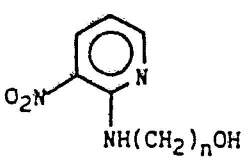

(IX)

en composé (X)

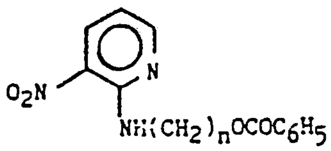

(X)

que l'on hydrogène en composé (XI)

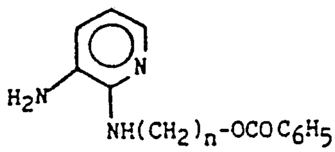

(XI)

on fait réagir ce dernier avec de l'urée pour obtenir le composé (XII)

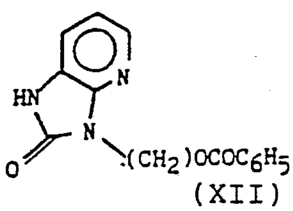

(XII)

que l'on hydrolyse en alcool (XIII)

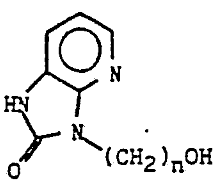

(XIII)

on transforme ce composé en dérivé de l'alcool (XIV)

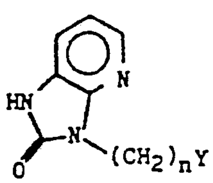

(XIV)

dans lequel Y est un groupe labile, et finalement on fait réagir ce composé avec un composé (II)

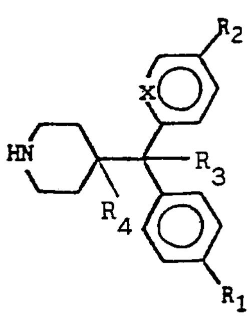

(II)

pour obtenir le composé (I).

4. Médicament caractérisé en ce qu'il contient un composé selon la revendication 1.

5. Composition pharmaceutique caractérisée en ce qu'elle contient un composé selon la revendication 1 en association avec tout excipient approprié.

Revendications pour les Etats contractants suivants : ES et GR.

1. Procédé de préparation de dérivés d'imidazo[4,5-b]pyridinone-2 répondant à la formule (I)

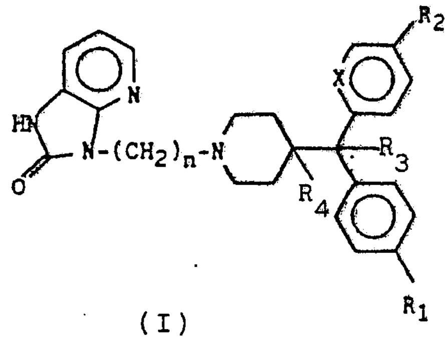

(I)

dans laquelle
n est 2, 3 ou 4,
X est CH ou N,
$R_1$ et $R_2$ représentent chacun, indépendamment l'un de l'autre, un atome d'hydrogène ou d'halogène ou un radical ($C_{1-4}$alcoxy et,
soit $R_3$ est H ou OH et $R_4$ est H, soit $R_3$ et $R_4$ forment une liaison,
ainsi que de leurs énantiomères et de leurs sels d'addition aux acides pharmaceutiquement acceptables, procédé caractérisé en ce que l'on fait réagir un composé (II)

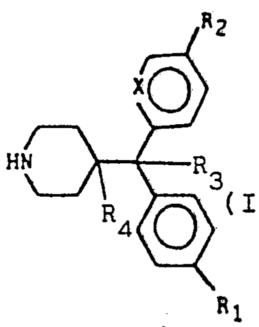

(II)

- soit avec un composé (III)

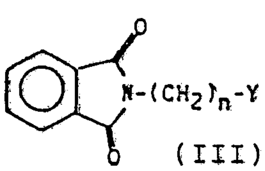

(III)

pour obtenir le composé (IV)

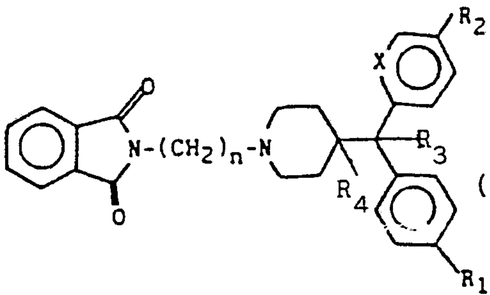

(IV)

que l'on fait réagir avec de l'hydrazine pour obtenir le composé (VI),
- soit avec de l'acrylonitrile ou un composé $Y-(CH_2)_{n-1}-CN$ dans lequel Y est un groupe labile, pour obtenir le composé (V)

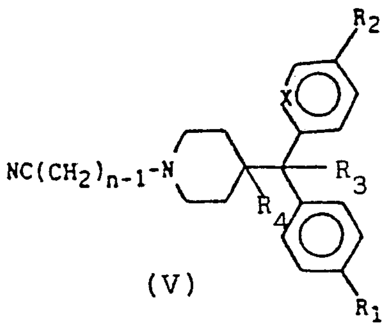

(V)

que l'on hydrogène, en présence de nickel de Raney, en composé (VI),

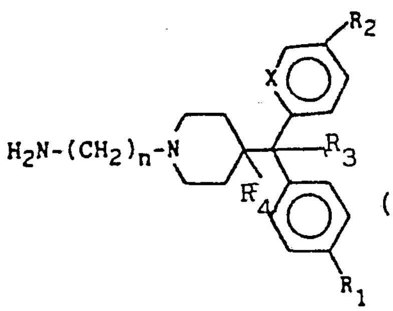

(VI)

et on fait alors réagir celui-ci avec la chloro-2 nitro-3 pyridine, en présence de carbonate de potassium, dans de l'éthanol, pour obtenir le composé (VII)

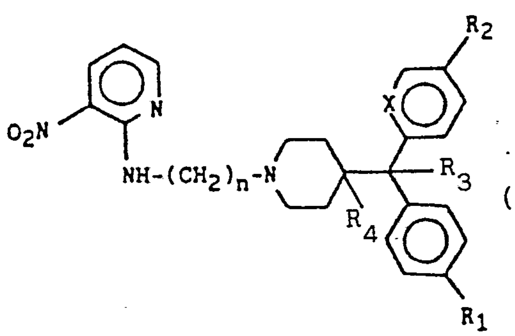

(VII).

que l'on hydrogène en composé (VIII)

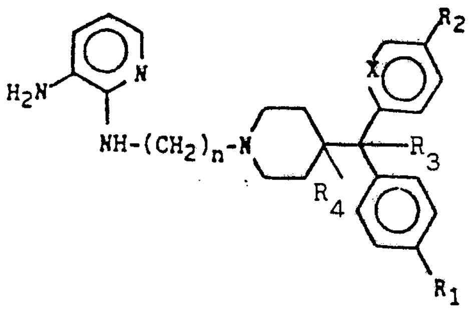

(VIII)

et enfin on fait réagir ce composé, par exemple, avec du pyrocarbonate d'éthyle dans du toluène pour obtenir le composé (I).

2. Procédé de préparation selon la revendication 1, procédé caractérisé en ce que l'on estérifie le composé (IX)

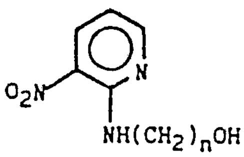

(IX)

en composé (X)

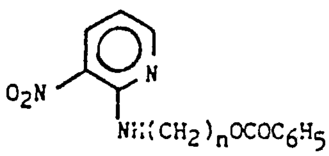

(X)

que l'on hydrogène en composé (XI)

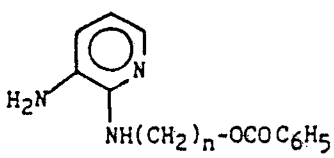

(XI)

on fait réagir ce dernier avec de l'urée pour obtenir le composé (XII)

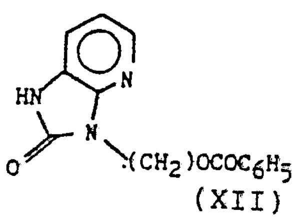

(XII)

que l'on hydrolyse en alcool (XIII)

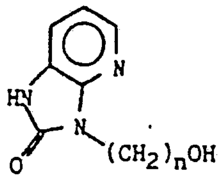 (XIII)

on transforme ce composé en dérivé de l'alcool (XIV)

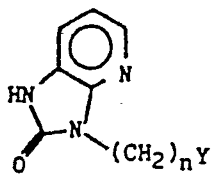

(XIV)

dans lequel Y est un groupe labile, et finalement on fait réagir ce composé avec un composé (II)

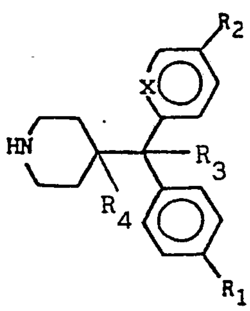 (II)

pour obtenir le composé (I).

Office européen des brevets

# RAPPORT DE RECHERCHE EUROPEENNE

Numero de la demande

EP 87 40 2222

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 181 793 (SYNTHELABO)<br>* Revendications *<br>----- | 1-5 | C 07 D 471/04<br>A 61 K 31/44 //<br>(C 07 D 471/04<br>C 07 D 235:00<br>C 07 D 221:00 ) |

| DOMAINES TECHNIQUES RECHERCHES (Int. Cl.4) |
|---|
| C 07 D 471/00 |

Le présent rapport a été établi pour toutes les revendications

| Lieu de la recherche | Date d'achèvement de la recherche | Examinateur |
|---|---|---|
| LA HAYE | 08-01-1988 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)